# EUROPEAN PATENT APPLICATION

(11) **EP 0 612 514 A1**
(43) Date of publication of application: **31.08.1994**
(21) Application number: 94300861.5
(22) Date of filing: 04.02.1994
(51) Int. Cl.: A61K 7/06

(54) **Method of making clear shampoo products**

(30) Priority: 24.02.1993 US 21821
(71) Applicant: DOW CORNING CORPORATION, Midland Michigan 48686-0994 (US)
(72) Inventor: Halloran, Daniel Joseph, Midland, Michigan 48642 (US)
(74) Representative: Kyle, Diana

(57) **Abstract**

A clear shampoo is described which contains a nonionic oil-in-water emulsion of a trimethylsilyl endblocked amine functional polydimethyl silicone. In the method of making clear and stable shampoo compositions, an emulsion of an amine functional siloxane is added to the shampoo composition without causing cloudiness and without contributing instability to the shampoo composition whereby phase separation occurs. In accordance with the method, an emulsion of an amine functional siloxane is added to a shampoo composition without the necessity of requiring that a pearling agent be employed for the purpose of disguising cloudiness.

## Description

This invention is directed to a clear shampoo and to clear shampoo products which contain a nonionic oil-in-water emulsion of an amine functional polydimethyl silicone.

Silicone containing nonionic emulsions have been traditionally known in the hair care art to tend towards the formation of surface creams, curds and separated oils, in anionic detergent systems such as shampoo products. Because of the positively charged nature of the silicone polymer at normal pH, such emulsions have not generally been compatible with anionic surfactants. These emulsions are particularly prone to form a surface cream when mixed into anionic detergent systems, resulting in unstable, unusable products.

The silicone containing nonionic emulsion is an excellent shampoo ingredient from the standpoint of being very substantive to hair and bringing about a dramatic improvement in both wet and dry combing. It is also effective in reducing water retention in hair which leads to shorter drying times. It is further capable of providing conditioning, such as improved luster and resistance to dry fly-away. Yet, formulators have heretofore marketed products which contain the nonionic emulsion in a form which contains a pearling agent, in order to mask any turbidity in the product.

A problem, therefore, exists in the personal care arena for clear shampoos containing nonionic silicone emulsions, which can be formulated into products without the necessity for masking or disguising otherwise turbid products or products having high cloud points.

This invention introduces a method of making clear and stable shampoo compositions in which an emulsion of an amine functional siloxane can be added to a shampoo composition without causing cloudiness and without contributing instability to the shampoo composition whereby phase separation occurs.

The invention further provides clear and stable shampoo compositions without the necessity of a pearling agent for the purpose of disguising the presence of cloudiness.

The conditioning agent which is used as a shampoo additive in the present invention, is a trimethylsilyl endblocked amine substituted siloxane polymer that is stabilized in the form of an aqueous emulsion by means of a nonionic surfactant. The siloxane polymer is represented by the formula: 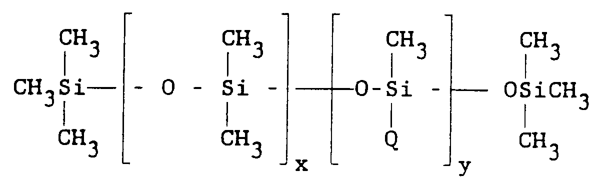
in which x and y are integers having positive values which control the molecular weight of the polymer. Typically, the integer x has a value of from one to ten thousand and the integer y has a value of from one to five hundred.

Q in the above formula represents an aminoalkyl group on silicon, such as the group -CH₂CH₂CH₂NHCH₂CH₂NH₂, although other equivalent aminoalkyl groups can be employed. Thus, Q can be for example other radicals such as -RNHCH₃; -RNHCH₂CH₂CH₂CH₃; -RN(CH₂CH₃)₂; -RNH(CH₂)₆NH₂; and -RNHCH₂CH₂CH₂N(CH₃)₂; in which R is a divalent alkylene radical having from three to six carbon atoms.

The adopted name of one silicone containing nonionic emulsion is Trimethylsilylamodimethicone (and) Octoxynol-40 (and) Isolaureth-6 (and) Propylene Glycol. This nomenclature is derived by The Cosmetic, Toiletry and Fragrance Association, of Washington, D.C., otherwise referred to as the CTFA.

Octoxynol-40 is a nonionic surfactant used to prepare such emulsions and is the CTFA designation for an ethoxylated alkyl phenol that conforms to the formula C₈H₁₇C₆H₄(OCH₂CH₂)ₙOH, in which n has an average value of about forty. This surfactant has an HLB value of 17.9 and is sold under the trademark TRITON® X-405 by the Union Carbide Chemical & Plastics Company, Industrial Chemicals Division, of Danbury, Connecticut USA.

Isolaureth-6 is the other nonionic surfactant used to prepare such emulsions and is the CTFA designation for a polyethylene glycol ether of a branched chain aliphatic twelve carbon atom alcohol conforming to the formula C₁₂H₂₅(OCH₂CH₂)ₙOH, in which n has an average value of six. This surfactant has an HLB value of 11.7 and is sold under the trademark TERGITOL® TMN-6 by the Union Carbide Chemical & Plastics Company, Industrial Chemicals Division, of Danbury, Connecticut USA.

Propylene Glycol is a dihydroxy alcohol or diol of the formula CH₃CHOHCH₂OH which functions as a freeze-thaw additive.

This nonionic silicone emulsion is available commercially as a product of the Dow Corning Corporation, Midland, Michigan USA. The silicone content of the emulsion is approximately thirty-five percent by weight.

Such emulsions are oil-in-water type compositions in which the siloxane polymer functions as the internal phase. The particle diameter of the siloxane polymer in the emulsion is typically of the order of magnitude of two hundred nanometers and above and therefore the emulsions are not microemulsions.

These emulsions have a milky white appearance to the naked eye, even at high dilutions of 0.1 weight percent. It is therefore very surprising that it is possible, in accordance with the present invention, to formulate clear shampoos from such silicone containing nonionic emulsions.

The shampoo composition prepared in accordance with the method of the present invention, contains other surfactants such as anionic, amphoteric, nonionic or cationic emulsifying agents and mixtures thereof. The shampoo surfactant should be capable of providing an acceptable level of foam on the hair and should also be capable of cleaning the hair.

Suitable anionic surfactants include sulfonated and sulfated alkyl, aralkyl and alkaryl anionic detergents such as alkyl succinates, alkyl sulfosuccinates and N-alkyl sarcosinates. Representative detergents are the sodium, magnesium, ammonium and the mono-, di- and triethanolamine salts of alkyl and aralkyl sulfates, as well as the salts of alkaryl sulfonates. The alkyl groups of the detergents should have a total of from twelve to twenty-one carbon atoms and may be unsaturated. Fatty alkyl groups are preferred. The sulfates may be sulfate ethers containing one to ten ethylene oxide or propylene oxide units per molecule, with two to three ethylene oxide units being sufficient for most purposes.

Typical anionic detergents are sodium lauryl sulfate, sodium lauryl ether sulfate, ammonium lauryl sulfate, triethanolamine lauryl sulfate, sodium C₁₄₋₁₆ olefin sulfonate, ammonium C₁₂₋₁₅ pareth sulfate, sodium myristyl ether sulfate, ammonium lauryl ether sulfate, disodium monooleamidosulfosuccinate, ammonium lauryl sulfosuccinate, sodium dodecylbenzene sulfonate, triethanolamine dodecylbenzene sulfonate and sodium N-lauryol sarcosinate.

Particularly suitable anionic surfactants are ammonium lauryl sulfate which is a product sold under the tradename STANDAPOL A and sodium laureth sulfate which is a product sold under the tradename STANDAPOL ES-3, by Henkel Corp./Emery Grp. Cospha/CD of Ambler, Pennsylvania. Sodium lauryl ether sulfate may also be employed and this product is sold under the tradename EMPICOL ESB 70 by Albright & Wilson Ltd. of Warley, United Kingdom.

Among the various surfactants classified as amphoteric or ampholytic which may be used are cocoamphocarboxyglycinate, cocoamphocarboxypropionate, cocobetaine, N-cocamidopropyldimethylglycine and N-lauryl-N-carboxymethyl-N-(2-hydroxyethyl) ethylenediamine. Other suitable amphoteric detergents which may be used include betaines and sultaines.

Betaines may have the formula R'R''R'''N⁺(CH₂)ₘCOO⁻ in which R' is an alkyl group having twelve to eighteen carbon atoms and mixtures thereof; R'' and R''' are lower alkyl groups of one to three carbon atoms; and m has a value of one to four. Specific compounds may include alpha-(tetradecyldimethylammonio)acetate, beta-(hexadecyldiethylammonio)propionate and gamma-(dodecyldimethylammonio)butyrate.

Sultaines may have the formula R'R''R'''N⁺(CH₂)ₘSO₃⁻ in which R', R'', R''' and m are the same as defined above. Specific compounds may include 3-(dodecyldimethylammonio)-propane-1-sulfonate and 3-(tetradecyldimethylammonio)ethane-1-sulfonate.

A suitable amphoteric surfactant for purposes of the present invention is cocamidopropyl betaine, which is a product sold under the tradename AMONYL 380BA by Seppic of Paris, France.

Nonionic surfactants suitable for use in the shampoo compositions of the present invention can be fatty acid alkanolamides and amine oxide surfactants. Representative fatty acid alkanolamides include fatty acid diethanolamides such as isostearic acid diethanolamide, lauric acid diethanolamide, capric acid diethanolamide, coconut fatty acid diethanolamide, linoleic acid diethanolamide, myristic acid diethanolamide, oleic acid diethanolamide and stearic acid diethanolamide. Suitable fatty acid monoethanolamides include coconut fatty acid monoethanolamide. Fatty acid monisopropanolamides which may be used are oleic acid monoisopropanolamide and lauric acid monoisopropanolamide.

Amine oxide nonionic surfactants suitable for use in the present invention are N-alkyl amine oxides such as N-cocodimethylamine oxide, N-lauryl dimethylamine oxide, N-myristyl dimethylamine oxide and N-stearyl dimethylamine oxide. Suitable N-acyl amine oxides are N-cocoamidopropyl dimethylamine oxide and N-tallowamidopropyl dimethylamine oxide. N-alkoxyalkyl amine oxides such as bis(2-hydroxyethyl) C₁₂₋₁₅ alkoxy-propylamine oxide may also be employed. The hydrophobic portion of the amine oxide surfactant should be provided by a fatty hydrocarbon chain of ten to twenty-one carbon atoms.

A particularly suitable nonionic surfactant is cocamide DEA which is a product sold under the tradename MONAMID 1159 by Mona Industries of Paterson, New Jersey. Linoleic diethanolamide may also be employed and is sold under the tradename EMPILAN 2125 by Albright & Wilson Ltd. of Warley, United Kingdom.

Other nonionic surfactants which may be used are cocamide MEA sold under the tradename ORAMIDE ML 115 by Seppic of Paris, France; and PEG-120 methylglucose dioleate sold under the tradename GLUCAMATE DOE 120 by Amerchol Corporation, Edison, New Jersey.

Cationic surfactants useful in the shampoo compositions of the present invention include compounds containing amino or quaternary ammonium hydrophilic moieties in the molecule which are positively charged, such as quaternary ammonium salts. Representative quaternary ammonium salts are ditallowdimethyl ammonium chloride, ditallowdimethyl ammonium methyl sulfate, dihexadecyl dimethyl ammonium chloride, di(hydrogenated tallow) dimethyl ammonium chloride, dioctadecyl dimethyl ammonium chloride, dieicosyl dimethyl ammonium chloride, didocosyl dimethyl ammonium chloride, di(hydrogenated tallow) dimethyl ammonium acetate, dihexadecyl dimethyl ammonium acetate, ditallow dipropyl ammonium phosphate, ditallow dimethyl ammonium nitrate, di(coconutalkyl) dimethyl ammonium chloride and stearyl dimethyl benzyl ammonium chloride.

The shampoo compositions of the invention may contain other adjuvants which provide a product aesthetically pleasant to the consumer; such as thickeners, perfumes, colorants, electrolytes, pH control agents, foam boosters and builders, foam stabilizers, antimicrobials, antioxidants, ultraviolet light absorbers and medicaments. Such adjuvants however must be carefully selected so as not to cause any turbidity in the final clear shampoo product.

Thickeners facilitate the hand application of the shampoo composition to the hair and are added in sufficient quantities to provide a more luxurious effect. Shampoo compositions with viscosities in the range of five hundred to fifteen thousand mm²/s (centistokes) measured at 25°C., are generally considered sufficient. Representative thickening agents are sodium alignate; gum arabic; guar gum; hydroxypropyl guar gum; cellulose derivatives such as methylcellulose, hydroxypropyl methylcellulose, hydroxyethylcellulose and hydroxypropylcellulose; starch and starch derivatives such as hydroxyethylamylose and starch amylose; locust bean gum; electrolytes such as sodium chloride and ammonium chloride; saccharides such as fructose and glucose; and derivatives of saccharides such as PEG-120 methyl glucose dioleate.

Only cosmetically acceptable perfumes and fragrances should be used to prepare the shampoo composition. Colorants may be added where it is desired to confer a clear hue to the composition.

An acid is employed to adjust the pH within the range of four to nine. Any water soluble carboxylic acid or mineral acid may be employed. Suitable compounds include mineral acids such as hydrochloric acid, sulfuric acid and phosphoric acid; monocarboxylic acids such as acetic acid, lactic acid and propionic acid; and polycarboxylic acids such as succinic acid, adipic acid and citric acid.

Additional conditioners, other than the nonionic silicone emulsion, may be added to the shampoo composition. Conditioners such as organic cationic conditioning agents provide more hair grooming. Such cationic conditioning agents include quaternary nitrogen derivatives of cellulose ethers; homopolymers of dimethyldiallyl ammonium chloride; copolymers of acrylamide and dimethyldiallyl ammonium chloride; homopolymers or copolymers derived from acrylic acid or methacrylic acid which contain cationic nitrogen functional groups attached to the polymer by ester or amide linkages; polycondensation products of N,N'-bis-(2,3-epoxypropyl)-piperazine or piperazine-bis-acrylamide and piperazine; and copolymers of vinylpyrrolidone and acrylic acid esters with quaternary nitrogen functionality. Specific materials include the various polyquats Polyquaternium-7, Polyquaternium-8, Polyquaternium-10, Polyquaternium-11 and Polyquaternium-23.

Cationic surfactants such as cetyl trimethylammonium chloride, cetyl trimethylammonium bromide and stearyl trimethylammonium chloride, may also be used as a cationic conditioning agent.

A preservative may be required and representative compounds which may be employed include formaldehyde, DMDM hydantoin, 5-bromo-5-nitro-1,3-dioxane, methyl paraben, propyl paraben, sorbic acid, diazolidinyl urea and imidazolidinyl urea.

Other adjuvants such as vitamins and therapeutic agents may be added to the shampoo compositions of the present invention, provided such adjuvants do not interfere with the clarity of the product.

Clear shampoos prepared in accordance with the teaching of the method of the present invention contain from 3-30 percent by weight of an anionic surfactant as measured on a solids basis; from 0.25-25.0 percent by weight of a nonionic silicone emulsion; water in an amount of 50-97 percent by weight; sufficient of an electrolyte to provide a shampoo product having a viscosity of from 500-15,000 mm²/s (centistokes); and a pH adjusting amount of an acid sufficient to provide a shampoo product with a pH of 4-7.

Clear shampoos are prepared by forming an anionic surfactant solution, adding the nonionic silicone emulsion to the anionic surfactant solution, adjusting the pH of the mixture to a value between 4-7 and adding electrolytes and any other desired adjuvants.

It is important to add the nonionic silicone emulsion to the anionic surfactant solution accompanied by sufficient agitation to provide for a thorough mixing of the ingredients; but not at a speed which would tend to whip the mixture causing the formation of a foam or an aerated solution. The nonionic silicone emulsion should be fed to the anionic surfactant solution at a rate which allows dispersement of the emulsion and at a temperature at which water is not evaporated. Temperatures of 35°C. or less have been found to be most suitable.

While foam boosters and thickeners may be added at any point in the process, these additional ingredients should be added during preparation of the anionic surfactant solution. If other anionic surfactants are required, such supplemental components should be added following addition of the electrolyte.

Where additional conditioning agents are necessary, beyond the conditioning provided by the nonionic silicone emulsion, these agents can be added at any point in the process; although for the best results, they should be added as the last ingredient.

The invention is illustrated in more detail by reference to the following examples and tables.

### Example I

Two shampoo base formulas were prepared and are shown in Table I. In Table I, "Premix 1" refers to an aqueous mixed clear solution containing 150 grams of ammonium lauryl sulfate (30% solids), 15 grams of Cocamide DEA and 325 grams of water. "Emulsion 1" in Table I refers to a trimethylsilyl endblocked amine substituted siloxane polymer stabilized in the form of an aqueous emulsion by means of a nonionic surfactant. The siloxane polymer had the formula: 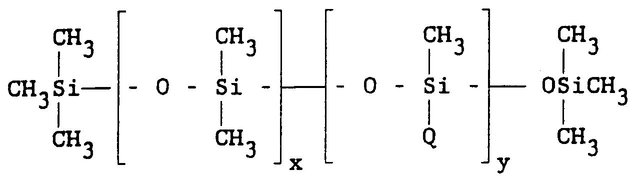
in which x had a value of 188 and y had a value of 10. Q was the aminoalkyl group -CH₂CH(CH₃)CH₂NHCH₂CH₂NH₂.

One nonionic surfactant used to prepare the emulsion was the ethoxylated alkyl phenol C₈H₁₇C₆H₄(OCH₂CH₂)₄₀OH and the other nonionic surfactant was the ethoxylated alkyl phenol C₈H₁₇C₆H₄(OCH₂CH₂)₉OH.

"Emulsion 2" in Table I was the same as "Emulsion 1", except that the integer x had a value of 274 and the integer y had a value of 24.

The two shampoo base formulas prepared in this example were tested visually with the naked eye and determined to be clear.

**TABLE I**

| | Shampoo Base Formula (grams) | |
|---|---|---|
| Ingredient | No. 1 | No. 2 |
| Premix 1 | 15.0 | 15.0 |
| Emulsion 1 | ---- | 1.0 |
| Emulsion 2 | 0.96 | ---- |
| Citric Acid (pH) | 5.5 | 5.5 |
| Ammonium Chloride | 0.09 | 0.12 |
| Visual | Clear | Clear |

The two shampoo base formulas were evaluated for their foam production and their wet feel and wet combining on hair tresses. Both formulas performed as shampoos in these subjective evaluations.

### Example II

In order to illustrate the importance of proper mixing of the emulsion into the shampoo base formula, an equivalent Shampoo Base Formula containing a cationic silicone emulsion was prepared at five different mixing speeds as shown in Table II. The "Mixing Setting" corresponded to the position of an air valve used to drive a standard laboratory mixer. A valve setting of 1/16 produced a very slow rate of rotation, whereas a setting of 1/2 produced an extremely rapid rate of rotation. As can be seen in Table II, hazy shampoos were produced at all rates except the rate corresponding to the "Mixing setting" of 3/8. The 3/8 setting produced a clear shampoo and was the rotational rate at which the silicone emulsion could be added without producing whipping, foaming or aeration of the mixture.

**TABLE II**

| Mixing Setting | Result (24 Hours) |
|---|---|
| 1/16 | Hazy |
| 1/8 | Hazy |
| 1/4 | Hazy |
| 3/8 | Clear |
| 1/2 | Hazy |

### Example III

In order to illustrate the importance of employing the proper processing temperature for preparation of the shampoo, an equivalent "Shampoo Base Formula " containing a cationic silicone emulsion was prepared at four different processing temperatures as shown in Table III. Table III reveals that low temperature processing is required to produce clear shampoos including the silicone emulsion. Thus, clear shampoos were produced at temperatures generally in the range of 9-34°C. Temperatures in the range of 5-30°C. are generally sufficient for purposes of the present invention.

**TABLE III**

| Process Temperature (°C.) | Result (24 Hours) |
|---|---|
| 9 | Clear |
| 21 | Essentially Clear |
| 34 | Hazy |
| 51 | Very Hazy |

Prior to the present invention, attempts by those skilled in the art to formulate clear shampoos containing nonionic silicone emulsions were unsuccessful. Yet, clear shampoos provide the consumer with many desired benefits, since such products imply a perception of purity, naturalness and health. Thus, a solution is clearly provided in the personal care arena by the use of our clear shampoo products.

Successful efforts by those skilled in the art to use silicone emulsions in a shampoo which contains a high level of anionic surfactant, have been hindered because such systems rapidly form insoluble creams, curds and separated oils. However, the present invention provides a viable approach to the production of clear shampoo products, without the formation of insoluble phases which would tend to interfere with overall product clarity.

## Claims

1. A method of making a clear shampoo composition containing a silicone nonionic emulsion comprising the steps of (i) forming an aqueous solution of an anionic surfactant, the anionic surfactant being present in an amount of from 3 to 30 percent by weight on a solids basis, based on the total weight of the clear shampoo composition; (ii) adding to the aqueous solution at a temperature of from 5 to 30°C. from 0.25 to 25 percent by weight based on the total weight of the clear shampoo composition of a silicone containing nonionic emulsion which is a trimethylsilyl endblocked amine substituted siloxane polymer stabilized in the form of an aqueous emulsion by means of at least two nonionic surfactants, the siloxane polymer being present in the nonionic emulsion in the form of particles having a diameter of at least about two hundred nanometers; (iii) mixing the solution containing the nonionic emulsion at a rate sufficient to disperse the siloxane polymer, but at a rate less than that which would cause the solution to foam or become aerated; (iv) adjusting the pH of the solution to a value of from 4 to 7; (v) and adding an electrolyte to the solution in an amount sufficient to provide a shampoo composition having a viscosity of from 500 to 15,000 mm²/s (centistokes); the total water content of the clear shampoo composition being from 50 to 97 percent by weight; steps (i) to (v) being carried out at temperatures of 35°C. or less.

2. A method according to claim 1 in which the electrolyte is a compound selected from the group consisting of sodium chloride and ammonium chloride.

3. A clear shampoo composition prepared in accordance with the method defined in claim 1.

4. A method of shampooing and conditioning hair comprising the steps of applying the clear shampoo composition defined in claim 1 to hair, rubbing the clear shampoo composition into the hair and removing the composition from the hair by rinsing the hair with water.

5. A method of making a clear shampoo composition containing a silicone nonionic emulsion comprising the steps of (i) forming an aqueous solution of an anionic surfactant, the anionic surfactant being present in an amount of from 3 to 30 percent by weight on a solids basis, based on the total weight of the clear shampoo composition; (ii) adding to the aqueous solution at a temperature of from 5 to 30°C. from 0.25 to 25 percent by weight based on the total weight of the clear shampoo composition of a silicone containing nonionic emulsion which is a trimethylsilyl endblocked amine substituted siloxane polymer stabilized in the form of an aqueous emulsion by means of a nonionic surfactant, (iii) mixing the solution containing the nonionic emulsion at a rate sufficient to disperse the siloxane polymer, but at a rate less than that which would cause the solution to foam or become aerated; and (iv) adjusting the pH of the solution to a value of from 4 to 7; steps (i) to (v) being carried out at temperatures of 35°C. or less.

6. A method according to claim 5 in which the silicone containing nonionic emulsion includes an ethoxylated alkyl phenol, a polyethylene glycol ether of a branched chain aliphatic alcohol and a dihydroxy alcohol.

7. A method according to claim 6 in which the dihydroxy alcohol is propylene glycol.
